# EUROPEAN PATENT SPECIFICATION

(11) **EP 4 056 177 B1**
(45) Date of publication and mention of the grant of the patent: **28.02.2024**
(21) Application number: 20896688.7
(22) Date of filing: 12.11.2020
(51) Int. Cl.: A61K 9/107, A61K 9/46, A61K 9/00, A61K 9/02, A61K 9/06, A61K 9/20, A61K 31/05, A61K 31/352, A61K 36/185, A61K 47/22, A61P 1/16, A61P 25/00, A61P 29/00, A61P 37/04, A61P 39/06, A61P 35/00

(54) **CANNABINOID NANOMICELLE PREPARATION AND METHOD FOR PREPARING SAME**
CANNABINOID-NANOMICELLENPRÄPARAT UND HERSTELLUNGSVERFAHREN DAFÜR
PRÉPARATION DE NANOMICELLES DE CANNABINOÏDE ET SON PROCÉDÉ DE PRÉPARATION

(30) Priority: 06.12.2019 CN 201911243459
(43) Date of publication of application: 14.09.2022
(73) Proprietor: Hanyi Biotechnology (Beijing) Co., Ltd., Beijing 100020 (CN)
(72) Inventor: TAN, Xin, Beijing 100020 (CN); WANG, Shubin, Beijing 100020 (CN); FAN, Dekai, Beijing 100020 (CN); SUN, Wuxing, Beijing 100020 (CN); XING, Junbo, Beijing 100020 (CN); ZHANG, Xuran, Beijing 100020 (CN)
(74) Representative: HGF
(86) International application number: PCT/CN2020/128307
(87) International publication number: WO 2021/109823

(56) References cited:
- EP-A1- 3 257 503
- WO-A1-2018/061007
- WO-A1-2018/150182
- WO-A1-2018/152334
- WO-A1-2019/008178
- WO-A1-2019/008179
- WO-A1-2019/036243
- WO-A1-2019/113685
- WO-A1-2020/002917
- CA-A1- 3 032 618
- CN-A- 101 686 946
- CN-A- 110 177 543
- US-A1- 2018 344 688
- LU, Bin: "Polymeric Micelles As Drug Carriers" In: "New Techniques and New Dosage forms of Drugs", 31 May 2005 (2005-05-31), People's Medical Publishing House, CN, XP009528584, ISBN: 7-117-06745-4 pages 63-72, * pp. 63-72 *
- FANG, Liang: "Vitamin E-TPGS" In: FANG, Liang: "Pharmaceutical Polymer Materials", 31 August 2015 (2015-08-31), China Medical Science and Technology Press, CN, XP009528585, ISBN: 978-7-5067-7419-2 pages 172-173, * pp. 172-173 *
- MENG, Shengnan: "Polymeric Micelles" In: "Pharmaceutics", 31 January 2016 (2016-01-31), China Medical Science and Technology Press, CN, XP009528586, ISBN: 978-7-5067-7881-7 pages 336-340, * pp. 336-340 *

## Description

### TECHNICAL FIELD

The invention relates to the technical field of pharmaceutical preparations, in particular to a cannabinoid nano-micelle preparation and a preparation method thereof, and particularly relates to a cannabinoid nano-micelle solid preparation and a preparation method thereof.

### BACKGROUND

Polymer micelles are core-shell type nanoparticles formed by self-assembly of a block copolymer in an aqueous solution. A hydrophobic drug can be embedded in a hydrophobic core, and a shell composed of hydrophilic chain segments forms a hydrated layer barrier to provide micelle stability. Its amphipathicity is realized by a hydrophilic block and a hydrophobic block. The hydrophilic block includes polyethylene glycol (PEG), polyethylene oxide (PEO) and polyvinylpyrrolidone (PVP); and the hydrophobic block includes polypropylene, polyamino acid, polylactic acid and the like. The polymer micelle is divided into an amphiphilic block copolymer, a triblock copolymer, a cross-linked copolymer and a graft copolymer. Polymer micelle solutions are mostly transparent solutions with a particle size of 10-100 nm, and a polymer micelle solution with a particle size of larger than 100 nm is opaque and is in an emulsion or suspension state. In the field of pharmaceutics, drug-loaded particles or drug nanocrystals with a size of 1-1000 nm are called nano-drugs.

Polymer nano-micelle drug loading has several unique advantages that: 1. the drug loading capacity is high, and the solubility of insoluble raw materials in water is greatly improved; 2. chemical structures of the raw materials can be solubilized without being changed; 3. slow release and controlled release of drugs can be realized, and stable blood concentration can be ensured in vivo for a long time; 4. the oral absorption of the drugs is promoted; 5. the targeting effect is achieved, and the small particle size is beneficial to penetrating into tumors with poor permeability; and 6. the lower CMC (critical micelle concentration) can be beneficial to keeping the micelle structure under the condition of being diluted by blood.

A nano-micelle preparation is generally a liquid oral preparation, particularly, almost no solid-state preparation exists in the fields of cannabinoid and monomer preparations, for example, the patent WO2019008178A1 describes a cannabinoid micelle liquid preparation.

WO2018152334A1 discloses compositions comprising cannabinoid and at least one surfactant such as poloxamer.

Cannabinoid is a fat-soluble substance and is almost insoluble in water, so that the development of cannabinoid in the field of preparations is greatly restricted. Cannabis preparations are mostly oil solutions which are inconvenient to take, poor in stability and low in absorption bioavailability. An aqueous solution preparation of cannabinoid contains nano-emulsion, nano-capsules, nano-microspheres, cyclodextrin inclusion and the like, the drug loading capacity is generally low and is about 0-3%, the stability in an aqueous solution is poor, and the stability in gastric juice is poor.

### SUMMARY

In order to overcome the defects in the prior art, the invention provides a cannabinoid nano-micelle preparation and a preparation method thereof.

Specifically, the cannabinoid nano-micelle preparation includes cannabinoid according to claim 1, an amphiphilic polymer according to claim 1, and optionally, a pharmaceutically acceptable freeze-drying protective additive and a pH regulator, and is prepared by a method including the following steps:
(1) preparing a cannabinoid nano-micelle solution from cannabinoid and an amphiphilic polymer;
(2) diluting the micellar solution obtained in the step (1), and drying to obtain cannabinoid nano-micelle powder; and
(3) preparing the cannabinoid nano-micelle powder obtained in the step (2) into the cannabinoid nano-micelle preparation,
wherein the drying in the step (2) is atomization freeze drying, wherein an atomization mode is selected from one or a combination of two or more of pneumatic atomization, pressure atomization, centrifugal atomization and ultrasonic atomization; and wherein the preparation is an inhalation preparation.

Specifically, in the above cannabinoid nano-micelle preparation, the content of the cannabinoid is 1-40% by weight (specifically, such as 1%, 5%, 10%, 15%, 20%, 25%, 30%, 35% and 40%).

Specifically, in the above cannabinoid nano-micelle preparation, the content of the amphiphilic polymer is 1-99% (specifically, such as 1%, 5%, 10%, 15%, 20%, 25%, 30%, 35%, 40%, 45%, 50%, 55%, 60%, 65%, 70%, 75%, 80%, 85%, 90%, 95% and 99%).

In one embodiment of the invention, the cannabinoid is selected from one or a combination of two or more of pure products of cannabidiol (CBD), cannabidivarin (CBDV), cannabigerol (CBG), cannabichromene (CBC), cannabinol (CBN), cannabidibutol (CBDB), cannabielsoin (CBE), cannabicyclol (CBL) and cannabinodiol (CBND), and the cannabinoid can be cannabinoid extracted from plants or synthetic cannabinoid, preferably the cannabinoid extracted from plants.

In another embodiment of the present invention, the cannabinoid is a cannabis extract, and includes one or a combination of two or more of CBD, CBDV, CBG, CBC, CBN, CBDB, CBE, CBL and CBND.

In one embodiment of the invention, the cannabinoid is CBD.

In one embodiment of the present invention, the amphiphilic polymer is vitamin E polyethylene glycol succinate (TPGS).

Specifically, the TPGS is selected from one or more of TPGS 200, TPGS 238, TPGS 400, TPGS 600, TPGS 1000, TPGS 2000, TPGS 3400, TPGS 3500, TPGS 4000 and TPGS 6000; and in one embodiment of the invention, the TPGS is the TPGS 1000.

In one embodiment of the present invention, the amphiphilic polymer is amphiphilic polyurethane.

Specifically, the amphiphilic polyurethane is an alternating copolymer which is obtained by alternately copolymerizing a hydrophilic chain segment, diisocyanate and the like, wherein the hydrophilic chain segment can be polyethylene glycol, polyoxyethylene and the like, and the diisocyanate is aliphatic or alicyclic diisocyanate, specifically, such as hexamethylene diisocyanate, trimethyl-1,6-hexamethylene diisocyanate, isophorone diisocyanate, cyclohexanedimethylene diisocyanate, 4,4-dicyclohexylmethane diisocyanate, 1,4-cyclohexane diisocyanate and the like.

Specifically, the amphiphilic polyurethane has a number-average molecular weight of 1000-50000 (specifically, such as 1000, 2000, 3000, 4000, 5000, 6000, 8000, 10000, 12000, 14000, 16000, 18000, 20000, 22000, 24000, 26000, 28000, 30000, 32000, 34000, 36000, 38000, 40000, 42000, 44000, 46000, 48000 and 50000).

In one embodiment of the invention, the hydrophilic chain segment in the amphiphilic polyurethane is polyethylene glycol, and the polyethylene glycol chain segment may have a number-average molecular weight of 200-12000 (specifically, such as 200, 400, 500, 600, 750, 1000, 2000, 4000, 5000, 6000, 8000, 10000 and 12000), in one embodiment of the invention, the polyethylene glycol chain segment may have a number-average molecular weight of 600-6000, and in one embodiment of the invention, the polyethylene glycol chain segment has a number-average molecular weight of 1000 or 1500.

In one embodiment of the present invention, the amphiphilic polymer is an amphiphilic polymer block copolymer, which is a block polymer composed of two or more polymers, and a hydrophilic part of the amphiphilic polymer can be polyethylene glycol, polyoxyethylene, povidone and the like, and a hydrophobic part of the amphiphilic polymer can be polyoxypropylene, polylactic acid, polystyrene, polycaprolactone, polyamino acid, poly(lactic-co-glycolic acid), polyacrylic acid and the like, such as one or a combination of two or more of poloxamer (PEO-PPO-PEO), a polylactic acid-polyethylene glycol-polylactic acid triblock copolymer (PLA-PEG-PLA), a polyethylene glycol-polyacrylic acid block copolymer (PEG-PAA), a polyethylene glycol-polyaspartic acid block copolymer (PEG-PASP), a polyethylene glycol-poly(lactic-co-glycolic acid) block copolymer (PEG-PLAG), a polyethylene glycol-polycaprolactone block copolymer (PEG-PCL), a polyethylene glycol-polylactic acid block copolymer (PEG-PLA/PTX), and a polyethylene glycol-polystyrene block copolymer (PEG-b-PS), and the like.

Specifically, the poloxamer is selected from one or a combination of two or more of poloxamer 188, poloxamer 338, poloxamer 407, poloxamer 124, poloxamer 215, poloxamer 237 and the like; and in one embodiment of the invention, the poloxamer is the poloxamer 188 or the poloxamer 338.

Specifically, in the polylactic acid-polyethylene glycol-polylactic acid triblock copolymer, a polyethylene glycol part may have a number-average molecular weight of 200-12000 (specifically, such as 200, 400, 500, 600, 750, 1000, 2000, 4000, 5000, 6000, 8000, 10000 and 12000), in one embodiment of the invention, the polyethylene glycol part may have a number-average molecular weight of 1000-6000, and in one embodiment of the invention, the polyethylene glycol part has a number-average molecular weight of 1000 or 2000; wherein a block ratio of PEG to LA is 1 :5-50 (a molar ratio) (specifically, such as 1:5, 1:10, 1:12, 1:15, 1:20, 1:25, 1:30, 1:40 and 1:50); and in one embodiment of the invention, the block ratio of PEG to LA is 1:10 or 1:20.

Specifically, the polyethylene glycol-polyacrylic acid block copolymer, the polyethylene glycol-polyaspartic acid block copolymer, the polyethylene glycol-poly(lactic-co-glycolic acid) block copolymer, the polyethylene glycol-polycaprolactone block copolymer, the polyethylene glycol-polylactic acid block copolymer and the polyethylene glycol-polystyrene block copolymer have a number-average molecular weight of 500-50000 (specifically, such as 500, 1000, 2000, 3000, 4000, 5000, 6000, 8000, 10000, 12000, 14000, 16000, 18000, 20000, 22000, 24000, 26000, 28000, 30000, 32000, 34000, 36000, 38000, 40000, 42000, 44000, 46000, 48000 and 50000); wherein polyethylene glycol is polyethylene glycol monomethyl ether, the polyethylene glycol part may have a number-average molecular weight of 200-12000 (specifically, such as 200, 400, 500, 600, 750, 1000, 2000, 4000, 5000, 6000, 8000, 10000 and 12000), in one embodiment of the invention, the polyethylene glycol part may have a number-average molecular weight of 1000-6000, and in one embodiment of the invention, the polyethylene glycol part has a number-average molecular weight of 1000, 2000 or 5000.

In one embodiment of the invention, the cannabinoid nano-micelle preparation further includes a pharmaceutically acceptable freeze-drying protective agent.

Specifically, in the above cannabinoid nano-micelle preparation, the content of the freeze-drying protective agent is 1-10% (specifically, such as 1%, 2%, 3%, 4%, 5%, 6%, 7%, 8%, 9% and 10%).

Specifically, the freeze-drying protective agent is selected from one or more of lactose, mannitol, sorbitol, cyclodextrin, hydroxypropyl-β-cyclodextrin, EDTA-2Na, trehalose, glucose, xylitol and maltose;

In one embodiment of the invention, the cannabinoid nano-micelle preparation further includes a pH regulator.

Specifically, in the above cannabinoid nano-micelle preparation, the content of the pH regulator is 0.01-10% (specifically, such as 0.01%, 0.05%, 0.1%, 0.2%, 0.3%, 0.4%, 0.5%, 1%, 2%, 3%, 4%, 5%, 6%, 7%, 8%, 9% and 10%).

Specifically, the pH regulator is selected from one or more of citric acid, sodium citrate, tartaric acid, sodium tartrate, acetic acid, sodium acetate, sodium dihydrogen phosphate, disodium hydrogen phosphate, hydrochloric acid, lactic acid and sodium hydroxide.

In one embodiment of the invention, the step (1) includes the following steps:
(1-1) adding raw material components of the cannabinoid nano-micelle preparation into a solvent for swelling;
(1-2) shearing the mixture obtained in the step (1-1) by using a high-shear dispersing emulsifier to obtain an emulsion; and
(1-3) stirring the emulsion obtained in the step (1-2) to obtain a clear transparent nano-micelle solution.

In one embodiment of the invention, the solvent in the step (1-1) is water, such as water for injection.

Specifically, the usage amount of the solvent in the step (1-1) is 2-10 times (a mass ratio, specifically, such as 2, 3, 4, 5, 6, 7, 8, 9 and 10 times) of that of the raw material components.

Specifically, the swelling in the step (1-1) is performed for 0.1-2h (specifically, such as 0.1h, 0.5h, 1h, 1.5h and 2h).

Specifically, the shearing in the step (1-2) is performed for 1-30 min (specifically, such as 1min, 5min, 10min, 15min, 20min, 25min and 30 min).

Specifically, in the step (1-3), the stirring is performed at 15-35°C (specifically, such as 15°C, 20°C, 25°C, 30°C and 35°C) for 1-48h (specifically, such as 1h, 6h, 12h, 24h, 36h and 48h).

Specifically, the cannabinoid nano-micelle solution has a particle size of 1-500 nm (specifically, such as 1 nm, 10 nm, 50 nm, 100 nm, 150 nm, 200 nm, 250 nm, 300 nm, 350 nm, 400 nm, 450 nm and 500 nm).

Specifically, a solvent for diluting in the step (2) is water, especially water for injection.

Specifically, a dilution ratio in the step (2) is 2-100 (specifically, such as 2, 5, 10, 20, 30, 40, 50, 60, 70, 80, 90 and 100).

Specifically, the drying in the step (2) is atomization freeze drying.

Specifically, the atomization freeze-drying includes the steps of atomization, freezing and drying; wherein an atomization mode is selected from one or a combination of two or more of pneumatic atomization, pressure atomization, centrifugal atomization and ultrasonic atomization; the freezing is performed at -10°C to -50°C (specifically, such as -10°C, -15°C, -20°C, -25°C, -30°C, -35°C, -40°C, -45°C and -50°C); and the drying step is performed under a vacuum degree of 40 Pa or below (specifically, such as 40 Pa, 30 Pa, 20 Pa and 10 Pa) at 10-35°C (specifically, such as 10°C, 15°C, 20°C, 25°C, 30°C and 35°C).

Specifically, the freeze drying includes the steps of pre-freezing, sublimation drying and desorption drying; wherein the pre-freezing is performed at -30°C to -50°C (specifically, such as -30°C, -35°C, -40°C, -45°C and -50°C) for 0.5-3 h (specifically, such as 0.5h, 1h, 2h and 3h) by controlling a vacuum degree to 1-100 Pa (1 Pa, 10 Pa, 20 Pa, 30 Pa, 40 Pa, 50 Pa, 60 Pa, 80 Pa and 100 Pa); the sublimation drying is performed at -20°C to 10°C (specifically, such as -20°C, -10°C, -5°C, 0°C, 5°C and 10°C) for 1-36h (specifically, such as 1h, 6h, 12h, 18h, 24h, 30h and 36h); and the desorption drying is performed at 10-30°C (specifically, such as 10°C, 15°C, 20°C, 25°C and 30°C) for 1-24h (specifically, such as 1h, 6h, 12h, 18h and 24h).

Specifically, the cannabinoid nano-micelle preparation is a solid preparation or a semi-solid preparation.

According to the claims, the cannabinoid nano-micelle preparation is an inhalation preparation, such as a dry powder inhalant and the like.

In one embodiment of the invention, the cannabinoid nano-micelle preparation is an inhalant, and has a particle size of 1-5 µm (specifically, such as 1 µm, 2 µm, 3 µm, 4 µm and 5 µm), wherein the drying in the step (2) is atomization freeze drying.

The preparation method according to claim 14 of the cannabinoid nano-micelle preparation provided by the invention includes the following steps:
(1) preparing a cannabinoid nano-micelle solution from cannabinoid and an amphiphilic polymer;
(2) diluting the micellar solution obtained in the step (1), and drying to obtain cannabinoid nano-micelle powder; and
(3) preparing the cannabinoid nano-micelle powder obtained in the step (2) into the cannabinoid nano-micelle preparation.

In one embodiment of the invention, the step (1) includes the following steps:
(1-1) adding raw material components of the cannabinoid nano-micelle preparation into a solvent for swelling;
(1-2) shearing the mixture obtained in the step (1-1) by using a high-shear dispersing emulsifier to obtain an emulsion; and
(1-3) stirring the emulsion obtained in the step (1-2) to obtain a clear transparent nano-micelle solution.

In one embodiment of the invention, the solvent in the step (1-1) is water, such as water for injection.

Specifically, the usage amount of the solvent in the step (1-1) is 2-10 times (a mass ratio, specifically, such as 2, 3, 4, 5, 6, 7, 8, 9 and 10 times) of that of the raw material components.

Specifically, the swelling in the step (1-1) is performed for 0.1-2h (specifically, such as 0.1h, 0.5h, 1h, 1.5h and 2h).

Specifically, the shearing in the step (1-2) is performed for 1-30 min (specifically, such as 1min, 5min, 10min, 15min, 20min, 25min and 30 min).

Specifically, in the step (1-3), the stirring is performed at 15-35°C (specifically, such as 15°C, 20°C, 25°C, 30°C and 35°C) for 1-48h (specifically, such as 1h, 6h, 12h, 24h, 36h and 48h).

Specifically, the cannabinoid nano-micelle solution has a particle size of 1-500 nm (specifically, such as 1 nm, 10 nm, 50 nm, 100 nm, 150 nm, 200 nm, 250 nm, 300 nm, 350 nm, 400 nm, 450 nm and 500 nm).

Specifically, a solvent for diluting in the step (2) is water, especially water for injection.

Specifically, a dilution ratio in the step (2) is 2-100 (specifically, such as 2, 5, 10, 20, 30, 40, 50, 60, 70, 80, 90 and 100).

Specifically, the drying in the step (2) is atomization freeze drying.

Specifically, the atomization freeze-drying includes the steps of atomization, freezing and drying; wherein an atomization mode is selected from one or a combination of two or more of pneumatic atomization, pressure atomization, centrifugal atomization and ultrasonic atomization; the freezing is performed at -10°C to -50°C (specifically, such as -10°C, -15°C, -20°C, -25°C, -30°C, -35°C, -40°C, -45°C and -50°C); and the drying step is performed under a vacuum degree of 40 Pa or below (specifically, such as 40 Pa, 30 Pa, 20 Pa and 10 Pa) at 10-35°C (specifically, such as 10°C, 15°C, 20°C, 25°C, 30°C and 35°C).

Specifically, the freeze drying includes the steps of pre-freezing, sublimation drying and desorption drying; wherein the pre-freezing is performed at -30°C to -50°C (specifically, such as -30°C, -35°C, -40°C, -45°C and -50°C) for 0.5-3 h (specifically, such as 0.5h, 1h, 2h and 3h) by controlling a vacuum degree to 1-100 Pa (1 Pa, 10 Pa, 20 Pa, 30 Pa, 40 Pa, 50 Pa, 60 Pa, 80 Pa and 100 Pa); the sublimation drying is performed at -20°C to 10°C (specifically, such as -20°C, -10°C, -5°C, 0°C, 5°C and 10°C) for 1-36h (specifically, such as 1h, 6h, 12h, 18h, 24h, 30h and 36h); and the desorption drying is performed at 10-30°C (specifically, such as 10°C, 15°C, 20°C, 25°C and 30°C) for 1-24h (specifically, such as 1h, 6h, 12h, 18h and 24h).

Disclosed, but not claimed, is a method for preventing and/or treating diseases, including the step of administering an effective amount of the cannabinoid nano-micelle preparation provided by the invention to a subject in need thereof.

Specifically, in the disclosed method, the cannabinoid nano-micelle preparation has the above definition of the present invention.

Specifically, in the disclosed method, the diseases are indications of the corresponding cannabinoid in the cannabinoid nano-micelle preparation; such as pain (such as chronic pain), inflammation (such as dermatitis), tumors (such as glioma, leukemia, prostate cancer and the like), liver injury (such as ischemic liver injury and liver injury caused by chronic alcoholism), nervous system diseases (such as epilepsy, multiple sclerosis, Parkinson's disease, Alzheimer's disease and the like) and the like (see for example, "Rong Guo, Xuan Chen, Hongyan Guo, Review on Pharmacological Effects of Tetrahydrocannabinol and Cannabidiol, Research and Development of Natural Products, 2017, 29: 449-1453").

In one embodiment of the invention, the subject is a mammal, in particular a human.

Specifically, the effective amount of the cannabinoid nano-micelle preparation depends on a plurality of factors including the age, weight, gender, natural health condition, and nutritional condition of patients, the taking time, a metabolic rate, severity of diseases, subjective judgment of diagnosis and treatment physicians and the like.

Disclosed, but not claimed, is a health-care product, including the cannabinoid nano-micelle preparation provided by the invention.

Disclosed, but not claimed, is a method for improving immunity and resisting oxidation, including the step of administering an effective amount of the health-care product to a subject in need thereof.

The cannabinoid nano-micelle preparation provided by the invention is high in effective component wrapping rate and transfer rate, high in drug loading capacity and high in stability, and a novel normal-temperature self-assembly technology is adopted, so that an active component cannabinoid is prevented from being degraded and discolored at high temperature; the bioavailability of the active ingredient is high, and a single dose can be reduced. Especially, the dry powder inhalant is high in drug loading capacity (10% or above), high in stability (the shelf life is 2-3 years), high in in-vitro deposition rate (an emptying rate can reach 95%, and an effective deposition rate can reach 45%), quick in inhalation effect and capable of providing continuous and stable blood concentration.

### DETAILED DESCRIPTION

Unless otherwise defined, all scientific and technical terms used in the invention have the same meaning as those generally understood by those skilled in the technical field to which the present invention relates, for example, the following abbreviations and corresponding substances appearing in the invention are as follows:
- CBDV: Cannabidivarin
- CBD: Cannabidiol
- CBG: Cannabigerol
- CBN: Cannabinol
- CBC: Cannabichromene
- CBDB: 4-butyl-5'-metyl-2'-(prop-1-en-2-yl)-1',2',3',4'-tetrahydro-(1,1'-bipheyl)-2,6-diol, Cannabidibutol
- CBE: Cannabielsoin
- CBL: Cannabicyclol
- CBND: Cannabinodiol
- TPGS: vitamin E polyethylene glycol succinate
- Poloxamer: Poloxamer
- PEG-PAA: Polyethylene glycol-polyacrylic acid block copolymer
- PEG-PASP: Polyethylene glycol-polyaspartic acid block copolymer
- PEG-PLAG: Polyethylene glycol-poly(lactic-co-glycolic acid) block copolymer
- PEG-PCL: Polyethylene glycol-polycaprolactone block copolymer
- PEG-PLA/PTX: Polyethylene glycol-polylactic acid block copolymer
- PEG-b-PS: Polyethylene glycol-polystyrene block copolymer
- SFD: Spray freeze drying

In the invention, the term 'cannabinoid' refers to a class of secondary metabolites containing a molecular structure of alkyl and a monoterpene group unique to cannabis plants, for example, CBD, CBDV, CBG, CBC, CBN, CBDB, CBE, CBL, CBND and the like (as described in "Xuan Chen, Ming Yang, Hongyan Guo, Research Advances in Cannabinoids of Cannabis sativa, Botanical Newton, 2011, 46 (2): 197-205 ").

In the invention, pure products of CBD, CBDV, CBG, CBC, CBN, CBDB, CBE, CBL and CBND refer to pure products of the compounds, especially corresponding commercially available products, and the purity of the compounds is at least 99.5% or above, especially 99.9% or above (the balance is impurities).

### Embodiment 1: Preparation of cannabinoid nano-micelle solution (not in the scope of the claims)

1. A specific formula of the cannabinoid nano-micelle solution is shown in the following table.

**Table 1 Formula of cannabinoid nano-micelle solution**

| F o r m u l a | CBD | TPGS 1000 | Poloxam er 188 | Polyure thane 4000 | PEG 2000-PAA 2000 | PEG 2000-PASP 1000 | PEG 1000-PLAG 3000 |
|---|---|---|---|---|---|---|---|
| 1 | 10% | 10% | 50% | 0 | 0 | 0 | 10% |
| 2 | 20% | 0 | 0 | 10% | 0 | 0 | 0 |
| 3 | 30% | 0 | 0 | 0 | 10% | 10% | 0 |
| 4 | 40% | 0 | 0 | 0 | 0 | 0 | 10% |
| 5 | 50% | 0 | 0 | 0 | 0 | 10% | 0 |
| 6 | 60% | 0 | 10% | 10% | 0 | 0 | 0 |

| F o r m u l a | PEG 1000-PCL 2000 | PEG 3400-PLA/PTX 4000 | PEG 5000-b-PS 5000 | Mannit ol | Sorbitol | Citric acid | Tartaric acid |
|---|---|---|---|---|---|---|---|
| 1 | 0 | 0 | 0 | 5% | 5% | 1% | 0 |
| 2 | 50% | 0 | 0 | 5% | 5% | 0 | 1% |
| 3 | 0 | 30% | 0 | 5% | 5% | 1% | 0 |
| 4 | 10% | 0 | 20% | 5% | 5% | 0 | 1% |
| 5 | 0 | 10% | 10% | 5% | 5% | 1% | 0 |
| 6 | 0 | 0 | 0 | 5% | 5% | 0% | 1% |

| F o r m u l a | Cyclodext rin | Hydroxypr opyl-β-cyclodextri n | EDTA-2Na | Trehalo se | Leucine | Lactose | |
|---|---|---|---|---|---|---|---|
| 1 | 0 | 5% | 0.1% | 1% | 0.5% | 2.4% | |
| 2 | 5% | 0 | 0 | 1% | 0 | 3% | |
| 3 | 0 | 5% | 0.1% | 1% | 0.5% | 2.4% | |
| 4 | 5% | 0 | 0 | 1% | 0 | 3% | |
| 5 | 0 | 5% | 0.1% | 1% | 0.5% | 2.4% | |
| 6 | 5% | 0 | 0 | 1% | 0 | 3% | |

2. Preparation process

### (1) A first set of preparation processes (process 1)

The cannabinoid nano-micelle solution is prepared by feeding according to the proportion in formulae 1-6, specifically as follows:
a. swelling: 100g of raw materials are taken according to the formula in Table 1, and added into 800g of water for swelling for 2h;
b. emulsifying: the obtained mixture after swelling is sheared for 30 min by using a high-shear dispersing emulsifier at 50 Hz; and
c. stirring: the temperature of the emulsification system is controlled at 30°C, and stirring is performed for 48h to obtain a clear transparent nano-micelle solution with a particle size of less than 500nm.

The quality comparison of the cannabinoid nano-micelle solution obtained by the above preparation process is shown in the following table.

**Table 2 Quality comparison of cannabinoid nano-micelle solution**

| Formula | Quality of corresponding solution | | | | |
|---|---|---|---|---|---|
| | Solution clarity | Solution particle size | Solution color | Effective component wrapping rate% | Effective ingredient transfer rate% |
| 1 | Clear and transparent | 0-500 nm | Colourless | 100% | 100% |
| 2 | Clear and transparent | 0-500 nm | Colourless | 100% | 100% |
| 3 | Milky white liquid | 0-500 nm | White | 90% | 100% |
| 4 | Clear and transparent | 0-500 nm | Colourless | 100% | 100% |
| 5 | Clear and transparent | 0-500 nm | Colourless | 100% | 100% |
| 6 | Milky white liquid | 0-500 nm | White | 90% | 100% |

### (2) A second set of preparation processes (process 2)

The cannabinoid nano-micelle solution is prepared by feeding according to the proportion in formulae 1-6, specifically as follows:
a. melting: TPGS 1000, Poloxamer 188, polyurethane 4000, PEG 2000-PAA 2000, PEG 2000-PASP 1000, PEG 1000-PLAG 3000, PEG 1000-PCL 2000, PEG 3400-PLA/PTX 4000 and PEG 5000-b-PS 5000 are added according to the formulae 1-6 in Table 1, and melted at 70°C, cannabinoid and a monomer thereof are added after melting, and uniform dispersing is performed to prepare a liquid 1;
b. dissolving: according to the formulas 1-6 in Table 1, mannitol, sorbitol, citric acid, tartaric acid, cyclodextrin, hydroxypropyl-β-cyclodextrin, EDTA-2Na, trehalose, leucine and lactose are dissolved with 8 times of water to prepare a liquid 2; and
c. mixing and dissolving: the liquid "1" and the liquid "2" are mixed and dissolved at 40°C, and slow stirring is performed for 12h until a particle size of the solution is less than 500nm.

The quality comparison of the cannabinoid nano-micelle solution obtained by the above preparation process is shown in the following table.

**Table 3 Quality comparison of cannabinoid nano-micelle solution**

| Formula | Quality of corresponding solution | | | | |
|---|---|---|---|---|---|
| | Solution clarity | Solution particle size | Solution color | Effective component wrapping rate% | Effective ingredient transfer rate% |
| 1 | Clear and transparent | 0-500 nm | Colourless | 100% | 97% |
| 2 | Clear and transparent | 0-500 nm | Colourless | 100% | 97% |
| 3 | Milky white liquid | 0-500 nm | White | 90% | 96% |
| 4 | Clear and transparent | 0-500 nm | Colourless | 100% | 97% |
| 5 | Clear and transparent | 0-500 nm | Colourless | 100% | 97% |
| 6 | Milky white liquid | 0-500 nm | White | 90% | 96% |

It can be seen from the results in Table 2 and Table 3 that under the same formula, the transfer rate of the cannabinoid nano-micelle prepared by the first set of processes is higher than that of the cannabinoid nano-micelle prepared by the second set of processes, which may be due to the melting step adopted in the second set of processes, which makes cannabinoid degrade.

### Embodiment 2: Preparation of cannabinoid nano-micelle powder

A drying mode for preparing micelle powder includes vacuum drying, atomization freeze drying, nano spray drying, supercritical fluid technology and freeze drying, the formula 1 prepared by the first set of processes in Embodiment 1 is prepared into cannabinoid nano-micelle powder by the above drying techniques, respectively, and a quality comparison study is carried out, and a specific drying mode comparison is shown in Table 4.

Atomization freeze drying: the cannabinoid nano-micelle solution is sprayed into an atomization freeze dryer to produce the cannabinoid micelle micropowder, and a specific process is as follows:
a. atomization:
   the nano-micelle solution prepared by the process 1 in Embodiment 1 is diluted to 100 times by using purified water, and subjected to atomization freeze drying by using an atomization freeze dryer with an atomization device adopting ultrasonic atomization.
b. freezing:
   the solid content is 40% when pneumatic atomization, pressure atomization and centrifugal atomization are adopted, and the solid content is 10% when ultrasonic atomization is adopted;
   Feeding flow rate: 10ml/min;
   Freezing temperature: -45°C;
c. drying:
   Vacuum degree: 40Pa or below;
   Drying temperature: 30°C.

A freeze-drying process: the cannabinoid nano-micelle solution is put into a freeze-drying oven for freezing at -40°C for 3h by controlling a vacuum degree to be less than 10Pa, wherein primary drying is performed at -10°C for 24h; and secondary drying is performed at 25°C for 12h; and the dried material is sieved and subjected to straightening granulating through No. 1-No.5 sieves.

The quality comparison of the powder prepared by the above drying processes is shown in the following table.

**Table 4 Quality comparison of powder obtained by different drying processes**

| Drying mode | Process controllabil ity | Powder hygroscopic! ty% | Solubility | Surface morpholo gy | Particle size distribution d (0.9) µm | In-vitro deposition determinati on | |
|---|---|---|---|---|---|---|---|
| | | | | | | Emp tyin g rate % | Dep ositi on rate % |
| Vacuum drying | Simple and controllabl e | 2.0% | the solution is clear | Needle-like | 50.1 µm | 75% | 5% |
| Atomizatio n freeze drying | Simple and controllabl e | 1.0% | the solution is clear | Spherical | 5 µm | 95% | 45% |
| Nano spray drying | Simple and controllabl e | 1.3% | the solution is clear | Flaky | 20 µm | 90% | 30% |
| Supercritic al fluid | Complicate d and uncontrolla ble | 2.0% | the solution is clear | Flaky | 15 µm | 89% | 29% |
| Freeze drying | Simple and controllabl e | 1.5% | the solution is clear | Flaky | 48 µm | 70% | 4% |
| A detection method of the above parameters is as follows: | | | | | | | |
| 1. Hygroscopicity: a certain amount of dry powder is weighed, and placed in a test tube (with an outer diameter of 50mm and a height of 15mm), the test tube which is opened is placed for 24h in an environment with a temperature of 25°C and a relative humidity of 70%, the test tube is weighed and a moisture absorption weight gain is calculated. | | | | | | | |
| 2. Solubility: 0.2 g of dry drug powder is taken, and put into a test tube, 5mL of pure water is added, oscillating is performed, and the dissolution condition of the dry drug powder is observed. If the dry drug powder can be dissolved to obtain a clear solution, the drug has good solubility and can be used as a pulmonary inhalation preparation, and if the dry drug powder cannot be dissolved, the drug has poor water solubility and is not suitable for being used as a pulmonary inhalation preparation. | | | | | | | |
| 3. Surface morphology: the contact points among spherical particles are minimum, the dispersibility is best, and more plane contact points exist among particles in the shapes of flakes, needles and the like, so that the flowability is relatively poor. | | | | | | | |
| 4. Particle size distribution: represented by d (0.9) µm, and the particle size of 90% of particles is less than this particle size. The particles of 1-5 µm can enter the lung through the respiratory airflow, and a good lung deposition rate is achieved. | | | | | | | |
| 5. In-vitro deposition rate determination: the in-vitro deposition rate determination is carried out by using NGI at 60L/min, including an emptying rate% and an effective deposition rate%, the emptying rate of a solid powder inhalation preparation is not lower than 90%, and the deposition rate is greater than 30%. | | | | | | | |

Result analysis: through the comparative analysis of several drying modes from the aspects of process controllability, powder hygroscopicity, solubility, surface morphology, particle size distribution, in-vitro deposition rate determination and other indicators, it is determined that atomization freeze drying has more advantages in inhalation preparation than other powders, and freeze-dried powder is more suitable for the preparation of other solid preparations.

### Embodiment 3: Cannabinoid nano-micelle inhalation preparation

The powder obtained by atomization freeze drying in Embodiment 2 has a particle size of 1-5 µm, the surface morphology is spherical, the fluidity is better, the emptying rate is 95%, the deposition rate is 45%, the powder is suitable for being used as inhalation powder, and the powder is filled into No.2-No.00 capsules, vesicles and reservoirs or can be directly used for administration by inhalation.

The bioavailability comparison between the cannabinoid nano-micelle inhalation powder and the micellar solution prepared according to the method described in the embodiments of the patent application WO2019008178A1 is shown in the following table.

**Table 5 Bioavailability comparison**

| Item | Bioavailability AUC |
|---|---|
| Inhalation powder prepared by this solution | 60% |
| Micellar solution prepared according to the patent WO2019008178A1 | 18% |

### Embodiment 4: Preparation of cannabinoid nano-micelle effervescent tablets (not in the scope of the claims)

1. Formula: cannabinoid micelle powder (powder obtained by a freeze drying process in Embodiment 2), DL-tartaric acid, citric acid, sodium bicarbonate, mannitol, lactose, aspartame, xylitol and polyethylene glycol. A specific formula is shown in the following table.

**Table 6 Formula of cannabinoid nano-micelle effervescent tablets**

| Form ula | Cannabi noid micelle powder | DL-tarta ric acid % | Citr ic acid % | Sodium bicarbon ate% | Mannit ol% | Lactos e% | Asparta me% | Xylit ol% | Polyethy lene glycol% |
|---|---|---|---|---|---|---|---|---|---|
| 1 | 10% | 20% | 5% | 25% | 5% | 20% | 1% | 5% | 9% |
| 2 | 20% | 20% | 5% | 25% | 0 | 15% | 1% | 5% | 9% |
| 3 | 30% | 20% | 5% | 25% | 0 | 10% | 1% | 4% | 5% |

| | | | | | | | | | |
|---|---|---|---|---|---|---|---|---|---|
| Specification: the tablet weight is 2g. | | | | | | | | | |

### 2. Preparation process

According to the formula in Table 6, the cannabinoid micelle powder, DL-tartaric acid, citric acid, mannitol and polyethylene glycol are prepared into particles 1 by using a fluidized bed or by wet granulation; sodium bicarbonate, aspartame, lactose, xylitol and polyethylene glycol are prepared into particles 2 by using a fluidized bed or by wet granulation; and the particles 1, the particles 2 and the cannabinoid micelle powder are uniformly mixed, and pressed into effervescent tablets, wherein the tablet weight is controlled to be 2g.

The quality comparison of the effervescent tablets of different formulas is shown in the following table.

**Table 7 Quality comparison of cannabinoid nano-micelle effervescent tablets**

| Formula | Disintegration time/min | Dispersion uniformity | Solution state after effervescence | Content stability |
|---|---|---|---|---|
| 1 | 2 | Qualified | Clear and transparent | The content is stable after tablets are subjected to accelerated test for 6 months |
| 2 | 3 | Qualified | Clear and transparent | The content is stable after tablets are subjected to accelerated test for 6 months |
| 3 | 5 | Qualified | Clear and transparent | The content is stable after tablets are subjected to accelerated test for 6 months |

Result analysis: the effervescent tablets all meet the requirements of the effervescent tablet quality standard, the effervescent tablets are quickly dissolved in water and convenient to use and carry, the dissolving speed of cannabinoid powder can be increased, and the effervescent solution is a cannabinoid nano-micelle solution which can be directly taken.

### Embodiment 5: Preparation of cannabinoid nano-micelle sustained release tablets (not in the scope of the claims)

1. Formula: cannabinoid micelle powder (powder obtained by a freeze drying process in Embodiment 2), hydroxypropyl methylcellulose, polyvinylpyrrolidone, ethyl cellulose, lactose, superfine silica powder and magnesium stearate. A specific formula is shown in the following table.

**Table 8 Formula of cannabinoid nano-micelle sustained release tablets**

| Form ula | Cannabin oid micelle powder | Hydroxypro pyl methylcellu lose | Polyvinylpyrroli done | Ethyl cellulo se | Lacto se | Superfi ne silica powder | Magnesi um stearate |
|---|---|---|---|---|---|---|---|
| 1 | 10% | 50% | 9% | 9% | 20% | 1% | 1% |
| 2 | 30% | 20% | 10% | 10% | 28% | 1% | 1% |
| 3 | 50% | 20% | 10% | 6% | 12% | 1% | 1% |

2. Preparation process: according to the formula in Table 8, hydroxypropyl methylcellulose, polyvinylpyrrolidone, ethyl cellulose, lactose and tartaric acid are added, and granulated with a certain amount of absolute ethyl alcohol, cannabinoid micelle powder, superfine silica powder and magnesium stearate are added, uniform mixing is performed, and the obtained mixture is pressed into 1g of sustained release tablets.

The quality comparison of the sustained-release tablets of different formulas is shown in the following table.

**Table 9 Quality comparison of cannabinoid nano-micelle sustained release tablets**

| Formula | Properties | Tablet weight variation | Hardness kg/cm² | In vitro dissolution | Stability |
|---|---|---|---|---|---|
| 1 | White to brown tablets | ±2.5% | 9.0 | Linear release with a cumulative release rate of 92% | Stable after being subjected to accelerated test for 6 months |
| 2 | White to brown tablets | ±2.0% | 9.2 | Linear release with a cumulative release degree of 90% | Stable after being subjected to accelerated test for 6 months |
| 3 | White to brown tablets | ±2.6% | 9.1 | Linear release with a cumulative release rate of 91% | Stable after being subjected to accelerated test for 6 months |
| In vitro dissolution: 6 cannabinoid nano-micelle sustained release tablets in a same batch are taken and put into a rotating basket, a rotating speed of the rotating basket is set to be 120rpm, | | | | | |
| the temperature is set to be 37.0±0.5°C, and the cumulative release rate of linear release of a sample within 12h is 90% or more by taking 1000mL of fresh degassed distilled water as a dissolution medium. | | | | | |

The properties, tablet weight variation, hardness, in vitro dissolution and stability of the sustained-release tablets in the formulae all meet the requirements, cannabinoid can be slowly released in the intestinal tract, cannabinoid nano-micelles can slowly release cannabinoid after entering the blood, the dual sustained-release effect is achieved, the cannabinoid can maintain stable blood concentration in vivo for a long time, and the sustained-release tablets take effect on chronic pains for a long time.

### Embodiment 6: Preparation of cannabinoid nano-micelle orally disintegrating tablets (not in the scope of the claims)

1. Formula: cannabinoid micelle powder (powder obtained by a freeze drying process in Embodiment 2), L-HPC, lactose, microcrystalline cellulose, pregelatinized starch, superfine silica powder and magnesium stearate. A specific formula is shown in the following table.

**Table 10 Formula of cannabinoid nano-micelle orally disintegrating tablets**

| Formul a | Cannabino id micelle powder | L-HP C | Lactos e | Microcrystalli ne cellulose | Pregelatiniz ed starch | Superfi ne silica powder | Magnesiu m stearate |
|---|---|---|---|---|---|---|---|
| 1 | 10% | 30 % | 30% | 19% | 10% | 0.5% | 0.5% |
| 2 | 30% | 30 % | 20% | 10% | 9% | 0.5% | 0.5% |
| 3 | 50% | 25 % | 15% | 5% | 4% | 0.5% | 0.5% |

### 2. Preparation process

The materials are prepared into granules through a wet method or one-step granulation, and the granules are pressed into 0.5 g of rapidly disintegrating tablets.

The quality comparison of the quick-release tablets of different formulas is shown in the following table.

**Table 11 Mass comparison of cannabinoid nano-micelle quick-release tablets**

| Formula | Properties | Disintegration time S | Tablet weight variation | Stability |
|---|---|---|---|---|
| 1 | White to brown tablets | 40S | ±2.5% | Stable after being subjected to accelerated test for 6 months |
| 2 | White to brown tablets | 45S | ±2.3% | Stable after being subjected to accelerated test for 6 months |
| 3 | White to brown tablets | 48S | ±2.2% | Stable after being subjected to accelerated test for 6 months |

The properties, tablet weight variation, disintegration time and stability of the orally disintegrating tablets of the three formulas all meet requirements, the orally disintegrating tablets can be rapidly disintegrated in the mouth and absorbed into blood under the tongue after being taken in the mouth, the first-pass effect is avoided, and the orally disintegrating tablets are convenient for patients with dysphagia to take.

### Embodiment 7: Preparation of cannabinoid nano-micelle gel (not in the scope of the claims)

1. Formula: cannabinoid micelle powder (powder obtained by a freeze drying process in Embodiment 2), carbomer, glycerol, propylene glycol, triethanolamine, EDTA-2Na and ethylparaben. A specific formula is shown in the following table.

**Table 12 Formula of cannabinoid nano-micelle gel**

| Formu la | Cannabino id micelle powder | Carbom er | Glycer ol | Propyle ne glycol | Triethanolami ne | EDT A-2Na | Ethylparab en |
|---|---|---|---|---|---|---|---|
| 1 | 10% | 10% | 47% | 28.9% | 3% | 0.1% | 1% |
| 2 | 30% | 12% | 30% | 23.9% | 3% | 0.1% | 1% |
| 3 | 50% | 13% | 20% | 11.9% | 4% | 0.1% | 1% |

2. Preparation process: according to the formula in Table 12, 100 times of water is added into carbomer for swelling, triethanolamine is added to form a gel matrix, then the cannabinoid micelle powder, glycerol, propylene glycol, EDTA-2Na and ethylparaben are added, and uniform stirring is performed to obtain the cannabinoid nano-micelle gel.

The mass comparison of gel of different formulas is shown in the following table.

**Table 13 Quality comparison of cannabinoid nano-micelle gel**

| Formul a | Properties | Uniformity | Stability |
|---|---|---|---|
| 1 | Colorless to brown paste solid | Evenly dispersed and delicate | Stable after being subjected to accelerated test for 6 months |
| 2 | Colorless to brown paste solid | Evenly dispersed and delicate | Stable after being subjected to accelerated test for 6 months |
| 3 | Colorless to brown paste solid | Evenly dispersed and delicate | Stable after being subjected to accelerated test for 6 months |

The properties, uniformity and stability of the gel in the three formulas meet the requirements, and the gel can be directly smeared on the affected part to achieve the effects of treating dermatitis and joint chronic pain, removing acnes and freckles and the like.

### Embodiment 8: Preparation of cannabinoid nano-micelle dropping pills (not in the scope of the claims)

1. Formula: cannabinoid micelle powder (powder obtained by a freeze drying process in Embodiment 2), polyethylene glycol 4000 and polyethylene glycol 6000. A specific formula is shown in the following table.

**Table 14 Formula of cannabinoid nano-micelle dropping pills**

| Formula | Cannabinoid micelle powder | Polyethylene glycol 4000% | Polyethylene glycol 6000% | Water% |
|---|---|---|---|---|
| 1 | 10% | 40% | 45% | 5% |
| 2 | 30% | 25% | 30% | 5% |
| 3 | 50% | 20% | 25% | 5% |

2. Preparation process: according to the formula in Table 14, the cannabinoid micelle powder, polyethylene glycol 4000, polyethylene glycol 6000 and purified water are heated at 70°C to be melted to prepare slurry 1, the condensation temperature of paraffin oil is controlled to be 10°C, the slurry 1 is put into a pill dropping machine, dropping is performed to obtain dropping pills of 50 mg/pill, and the paraffin oil on the surfaces of the dropping pills is removed by using oil absorption cotton to obtain the cannabinoid nano-micelle dropping pills.

The quality comparison of the dropping pills of different formulas is shown in the following table.

**Table 15 Quality comparison of cannabinoid nano-micelle dropping pills**

| Formula | Properties | Pill weight difference | Disintegration time/min | Stability |
|---|---|---|---|---|
| 1 | White to yellowish-brown pills | ±8% | 10min | Stable after being subjected to accelerated test for 6 months |
| 2 | White to yellowish-brown pills | ±7% | 9min | Stable after being subjected to accelerated test for 6 months |
| 3 | White to yellowish-brown pills | ±8% | 11min | Stable after being subjected to accelerated test for 6 months |

The properties, pill weight difference, disintegration time and stability of the dropping pills in the three formulas all meet the requirements, and the dropping pills can be taken orally or sublingually, and is quick in effect and lasting in blood concentration.

### Embodiment 9: Preparation of cannabinoid nano-micelle suppository (not in the scope of the claims)

1. Formula: cannabinoid micelle powder (powder obtained by a freeze drying process in Embodiment 2), cocoa butter, mixed fatty acid glyceride, polyethylene glycol, and ethylparaben. A specific formula is shown in the following table.

**Table 16 Formula of cannabinoid nano-micelle suppository**

| Formula | Cannabinoid micelle powder | Cocoa butter | Mixed fatty acid glyceride | Polyethylene glycol | Ethylparaben |
|---|---|---|---|---|---|
| 1 | 10% | 50% | 30% | 9.5% | 0.5% |
| 2 | 30% | 40% | 20% | 9.5% | 0.5% |
| 3 | 50% | 25% | 20% | 4.5% | 0.5% |

2. Preparation process: according to the formula in the above table, the cannabinoid micelle powder, cocoa butter, mixed fatty acid glyceride, polyethylene glycol and ethylparaben are melted at 40-60°C, and the melted material is put into a suppository preparation machine to prepare suppositories such as spherical suppositories, bullet suppositories, capsule suppositories and the like.

The quality comparison of the suppositories of different formulas is shown in the following table.

**Table 17 Quality comparison of cannabinoid nano-micelle suppositories**

| Formula | Properties | Weight difference% | Melting time/min | Stability |
|---|---|---|---|---|
| 1 | White to brown suppository | 4.1% | 25min | Stable after being subj ected to accelerated test for 6 months |
| 2 | White to brown suppository | 4.0% | 26 min | Stable after being subj ected to accelerated test for 6 months |
| 3 | White to brown suppository | 4.5% | 27 min | Stable after being subj ected to accelerated test for 6 months |

The properties, the weight difference, the melting time and the stability of the suppositories in the three formulas all meet the requirements, and the suppositories can directly reach focuses to achieve the anti-inflammatory effect or be used for systemic administration to treat chronic pains, so that the application range of cannabinoid is expanded.

## Claims

1. A cannabinoid nano-micelle preparation, comprising cannabinoid and an amphiphilic polymer; wherein the content of the cannabinoid is 1-40% by weight, and the content of the amphiphilic polymer is 1-99%, and a preparation method of the preparation comprises the following steps:
(1) preparing a cannabinoid nano-micelle solution from cannabinoid and an amphiphilic polymer;
(2) diluting the micellar solution obtained in the step (1), and drying to obtain cannabinoid nano-micelle powder; and
(3) preparing the cannabinoid nano-micelle powder obtained in the step (2) into the cannabinoid nano-micelle preparation;
wherein the amphiphilic polymer is selected from one or a combination of two or more of vitamin E polyethylene glycol succinate, amphiphilic polyurethane, poloxamer, a polylactic acid-polyethylene glycol-polylactic acid triblock copolymer, a polyethylene glycol-polyacrylic acid block copolymer, a polyethylene glycol-polyaspartic acid block copolymer, a polyethylene glycol-poly(lactic-co-glycolic acid) block copolymer, a polyethylene glycol-polycaprolactone block copolymer, a polyethylene glycol-polylactic acid block copolymer and a polyethylene glycol-polystyrene block copolymer;
wherein the drying in the step (2) is atomization freeze drying, wherein an atomization mode is selected from one or a combination of two or more of pneumatic atomization, pressure atomization, centrifugal atomization and ultrasonic atomization; and wherein the preparation is an inhalation preparation.

2. The cannabinoid nano-micelle preparation according to claim 1, **characterized in that** the cannabinoid is selected from one or a combination of two or more of pure products of cannabidiol (CBD), cannabidivarin (CBDV), cannabigerol (CBG), cannabichromene (CBC), cannabinol (CBN), cannabidibutol (CBDB), cannabielsoin (CBE), cannabicyclol (CBL) and cannabinodiol (CBND); or,
the cannabinoid is a cannabis extract, and comprises one or a combination of two or more of CBD, CBDV, CBG, CBC, CBN, CBDB, CBE, CBL and CBND.

3. The cannabinoid nano-micelle preparation according to claim 1, **characterized in that** the vitamin E polyethylene glycol succinate is selected from one or more of TPGS 200, TPGS 238, TPGS 400, TPGS 600, TPGS 1000, TPGS 2000, TPGS 3400, TPGS 3500, TPGS 4000 and TPGS 6000.

4. The cannabinoid nano-micelle preparation according to claim 1, **characterized in that** the amphiphilic polyurethane is obtained by alternately copolymerizing a polyethylene glycol chain segment and a raw material comprising diisocyanate; wherein the amphiphilic polyurethane has a number-average molecular weight of 1000-50000, and the polyethylene glycol chain segment has a number-average molecular weight of 200-12000; and the diisocyanate is selected from the group consisting of hexamethylene diisocyanate, trimethyl-1,6-hexamethylene diisocyanate, isophorone diisocyanate, cyclohexanedimethylene diisocyanate, 4,4-dicyclohexylmethane diisocyanate, and 1,4-cyclohexane diisocyanate.

5. The cannabinoid nano-micelle preparation according to claim 1, **characterized in that** the poloxamer is selected from one or a combination of two or more of poloxamer 188, poloxamer 338, poloxamer 407, poloxamer 124, poloxamer 215 and poloxamer 237;
in the polylactic acid-polyethylene glycol-polylactic acid triblock copolymer, a polyethylene glycol part has a number-average molecular weight of 200-12000, wherein a block ratio of PEG to LA is 1:(5-50); and
the polyethylene glycol-polyacrylic acid block copolymer, the polyethylene glycol-polyaspartic acid block copolymer, the polyethylene glycol-poly(lactic-co-glycolic acid) block copolymer, the polyethylene glycol-polycaprolactone block copolymer, the polyethylene glycol-polylactic acid block copolymer and the polyethylene glycol-polystyrene block copolymer have a number-average molecular weight of 500-50000, wherein polyethylene glycol is polyethylene glycol monomethyl ether, and the polyethylene glycol part has a number-average molecular weight of 200-12000.

6. The cannabinoid nano-micelle preparation according to claim 1, **characterized in that** the cannabinoid nano-micelle preparation further comprises a pharmaceutically acceptable freeze-drying protective agent, wherein the content of the freeze-drying protective agent is 1-10%, and the freeze-drying protective agent is selected from one or more of lactose, mannitol, sorbitol, cyclodextrin, hydroxypropyl-β-cyclodextrin, EDTA-2Na, trehalose, glucose, xylitol and maltose.

7. The cannabinoid nano-micelle preparation according to claim 6, **characterized in that** the cannabinoid nano-micelle preparation further comprises a pH regulator, wherein the content of the pH regulator is 0.01-10%, and the pH regulator is selected from one or more of citric acid, sodium citrate, tartaric acid, sodium tartrate, acetic acid, sodium acetate, sodium dihydrogen phosphate, disodium hydrogen phosphate, hydrochloric acid, lactic acid and sodium hydroxide.

8. The cannabinoid nano-micelle preparation according to any one of claims 1-7, **characterized in that** the step (1) comprises the following steps:
(1-1) adding raw material components of the cannabinoid nano-micelle preparation into a solvent for swelling;
(1-2) shearing the mixture obtained in the step (1-1) by using a high-shear dispersing emulsifier to obtain an emulsion; and
(1-3) stirring the emulsion obtained in the step (1-2) to obtain a clear transparent nano-micelle solution.

9. The cannabinoid nano-micelle preparation according to claim 8, **characterized in that** a mass ratio of the solvent to the raw material components in the step (1-1) is (2-10):1;
the swelling in the step (1-1) is performed for 0.1-2h;
the shearing in the step (1-2) is performed for 1-30 min; and
the stirring in the step (1-3) is performed at 15-35°C.

10. The cannabinoid nano-micelle preparation according to claim 8, **characterized in that** the cannabinoid nano-micelle solution has a particle size of 1-500 nm.

11. The cannabinoid nano-micelle preparation according to any one of claims 1-7, **characterized in that** a dilution ratio in the step (2) is 2-100.

12. The cannabinoid nano-micelle preparation according to claim 1, **characterized in that** the preparation is a solid preparation or a semi-solid preparation.

13. The cannabinoid nano-micelle preparation according to claim 1, **characterized in that** the preparation is a dry powder inhalant.

14. A preparation method of the cannabinoid nano-micelle preparation according to any one of claims 1-13, comprising the following steps:
(1) preparing a cannabinoid nano-micelle solution from cannabinoid and an amphiphilic polymer;
(2) diluting the micellar solution obtained in the step (1), and drying to obtain cannabinoid nano-micelle powder; and
(3) preparing the cannabinoid nano-micelle powder obtained in the step (2) into the cannabinoid nano-micelle preparation;
preferably, the step (1) comprises the following steps:
(1-1) adding raw material components of the cannabinoid nano-micelle preparation according to any one of claims 1-9 into a solvent for swelling;
(1-2) shearing the mixture obtained in the step (1-1) by using a high-shear dispersing emulsifier to obtain an emulsion; and
(1-3) stirring the emulsion obtained in the step (1-2) to obtain a clear transparent nano-micelle solution.

## Patentansprüche

1. Cannabinoid-Nano-Mizellen-Zubereitung, die ein Cannabinoid und ein amphiphiles Polymer umfasst; wobei der Anteil des Cannabinoids 1-40 Gew.-% beträgt, und wobei der Anteil des amphiphilen Polymers 1-99% beträgt, und wobei ein Zubereitungsverfahren für die Zubereitung die folgenden Schritte umfasst:
(1) Zubereiten einer Cannabinoid-Nano-Mizellen-Lösung aus Cannabinoid und einem amphiphilen Polymer;
(2) Verdünnen der in Schritt (1) erhaltenen Mizellenlösung und Trocknen, um Cannabinoid-Nano-Mizellen-Pulver zu erhalten; und
(3) Zubereiten des in Schritt (2) erhaltenen Cannabinoid-Nano-Mizellen-Pulvers als die Cannabinoid-Nano-Mizellen-Zubereitung; wobei das amphiphile Polymer ausgewählt ist aus einem oder einer Kombination aus zwei oder mehreren der folgenden: Vitamin E-Polyethylenglycolsuccinat, amphiphilem Polyurethan, Poloxamer, einem Polymilchsäure-Polyethylenglycol-Polymilchsäure-Triblock-Copolymer, einem Polyethylenglycol-Polyacrylsäure-Block-Copolymer, einem Polyethylenglycol-Polyasparaginsäure-Block-Copolymer, einem Polyethylenglycolpoly(lactid-co-glycolsäure)-Block-Copolymer, einem Polyethylenglycolpolycaprolacton-Block-Copolymer, einem Polyethylenglycol-Polymilchsäure-Block-Copolymer und einem Polyethylenglycol-Polystyrol-Block-Copolymer; wobei es sich bei dem Trocknen in Schritt (2) um Zerstäubungs-Gefriertrocknen handelt, wobei ein Zerstäubungsmodus ausgewählt wird aus einem oder einer Kombination aus zwei oder mehreren der folgenden: pneumatische Zerstäubung, Druckzerstäubung, zentrifugale Zerstäubung und Zerstäubung durch Ultraschall; und wobei es sich bei der Zubereitung um eine Inhalationszubereitung handelt.

2. Cannabinoid-Nano-Mizellen-Zubereitung nach Anspruch 1, **dadurch gekennzeichnet, dass** das Cannabinoid ausgewählt ist aus einem oder einer Kombination aus zwei oder mehreren der folgenden: reine Produkte aus Cannabidiol (CBD), Cannabidivarin (CBD V), Cannabigerol (CBG), Cannabichromene (CBC), Cannabinol (CBN), Cannabidibutol (CBDB), Cannabielsoin (CBE), Cannabicyclol (CBL) und Cannabinodiol (CBND); oder es handelt sich bei dem Cannabinoid um ein Cannabisextrakt, und es umfasst einen oder eine Kombination aus zwei oder mehreren der folgenden: CBD, CBDV, CBG, CBC, CBN, CBDB, CBE, CBL und CBND.

3. Cannabinoid-Nano-Mizellen-Zubereitung nach Anspruch 1, **dadurch gekennzeichnet, dass** das Vitamin E-Polyethylenglycolsuccinat ausgewählt ist aus einem oder mehreren der folgenden: TPGS 200, TPGS 238, TPGS 400, TPGS 600, TPGS 1000, TPGS 2000, TPGS 3400, TPGS 3500, TPGS 4000 und TPGS 6000.

4. Cannabinoid-Nano-Mizellen-Zubereitung nach Anspruch 1, **dadurch gekennzeichnet, dass** das amphiphile Polyurethan erhalten wird durch wechselweise Copolymerisation eines Segments der Polyethylenglycolkette und eines Rohstoffs, der Diisocyanat umfasst; wobei das amphiphile Polyurethan ein zahlengemitteltes Molekulargewicht von 1000-50000 aufweist, und wobei das Segment der Polyethylenglycolkette ein zahlengemitteltes Molekulargewicht 200-12000 aufweist; und wobei das Diisocyanat ausgewählt ist aus der Gruppe bestehend aus: Hexamethylendiisocyanat, Trimethyl-1,6-hexamethylendiisocyanat, IsophoronDiisocyanat, Cyclohexandimethylen-Diisocyanat, 4,4-Dicyclohexylmethan-Diisocyanat, und 1,4-Cyclohexan-Diisocyanat.

5. Cannabinoid-Nano-Mizellen-Zubereitung nach Anspruch 1, **dadurch gekennzeichnet, dass** das Poloxamer ausgewählt ist aus einem oder aus einer Kombination aus zwei oder mehreren der folgenden: Poloxamer 188, Poloxamer 338, Poloxamer 407, Poloxamer 124, Poloxamer 215 und Poloxamer 237;
wobei in dem Polymilchsäure-Polyethylenglycol-Polymilchsäure-Triblock-Copolymer ein Polyethylenglycolanteil ein zahlengemitteltes Molekulargewicht von 200-12000 aufweist, wobei ein Blockverhältnis von PEG zu LA 1:(5-50) beträgt, und
wobei das Polyethylenglycol-Polyacrylsäure-Block-Copolymer, das Polyethylenglycol-Polyasparaginsäure-Block-Copolymer, das Polyethylenglycolpoly(lactid-co-glycolsäure)-Block-Copolymer, das Polyethylenglycolpolycaprolacton-Block-Copolymer, das Polyethylenglycol-Polymilchsäure-Block-Copolymer und das Polyethylenglycol-Polystyrol-Block-Copolymer ein zahlengemitteltes Molekulargewicht von 500-50000 aufweisen, wobei dass Polyethylenglycol Polyethylenglycol-Monomethylether ist, und wobei der Polyethylenglycolanteil ein zahlengemitteltes Molekulargewicht von 200-12000 aufweist.

6. Cannabinoid-Nano-Mizellen-Zubereitung nach Anspruch 1, **dadurch gekennzeichnet, dass** die Cannabinoid-Nano-Mizellen-Zubereitung ferner eine pharmazeutisch akzeptable Gefriertrocknungsschutzsubstanz umfasst, wobei der Anteil der Gefriertrocknungsschutzsubstanz 1-10% beträgt, und wobei die Gefriertrocknungsschutzsubstanz ausgewählt ist aus einem oder mehreren der folgenden: Lactose, Mannitol, Sorbitol, Cyclodextrin, Hydroxypropyl-β-Cyclodextrin, EDTA-2Na, Trehalose, Glucose, Xylitol und Maltose.

7. Cannabinoid-Nano-Mizellen-Zubereitung nach Anspruch 6, **dadurch gekennzeichnet, dass** die Cannabinoid-Nano-Mizellen-Zubereitung ferner einen pH-Regler umfasst, wobei der Anteil des pH-Reglers 0,01-10% beträgt, und wobei der pH-Regler ausgewählt ist aus einem oder mehreren der folgenden: Zitronensäure, Natriumcitrat, Weinsäure, Natriumtartrat, Essigsäure, Natriumacetat, Natriumdihydrogenphosphat, Dinatriumwasserstoffphosphat, Salzsäure, Milchsäure und Natriumhydroxid.

8. Cannabinoid-Nano-Mizellen-Zubereitung nach einem der Ansprüche 1-7, **dadurch gekennzeichnet, dass** der Schritt (1) die folgenden Schritte umfasst:
(1-1) Hinzufügen von Rohstoffkomponenten der Cannabinoid-Nano-Mizellen-Zubereitung in ein Lösungsmittel zum Quellen;
(1-2) Scheren des in dem Schritt (1-1) erhaltenen Gemischs unter Verwendung eines Hochscher-Dispersionsemulgators, um eine Emulsion zu erhalten; und
(1-3) Rühren der in dem Schritt (1-2) erhaltenen Emulsion, um eine klare, transparente Nano-Mizellen-Lösung zu erhalten.

9. Cannabinoid-Nano-Mizellen-Zubereitung nach Anspruch 8, **dadurch gekennzeichnet, dass** ein Massenverhältnis des Lösungsmittels zu den Rohstoffkomponenten in dem Schritt (1-1) (2-10): 1 beträgt;
wobei das Quellen in dem Schritt (1-1) über 0,1-2h ausgeführt wird;
wobei das Scheren in dem Schritt (1-2) über 1-30 Minuten ausgeführt wird; und
wobei das Rühren in dem Schritt (1-3) bei 15-35 °C ausgeführt wird.

10. Cannabinoid-Nano-Mizellen-Zubereitung nach Anspruch 8, **dadurch gekennzeichnet, dass** die Cannabinoid-Nano-Mizellen-Lösung eine Partikelgröße von 1-500 nm aufweist.

11. Cannabinoid-Nano-Mizellen-Zubereitung nach einem der Ansprüche 1-7, **dadurch gekennzeichnet, dass** das Verdünnungsverhältnis in Schritt (2) 2-100 beträgt.

12. Cannabinoid-Nano-Mizellen-Zubereitung nach Anspruch 1, **dadurch gekennzeichnet, dass** die Zubereitung eine feste oder halbfeste Zubereitung ist.

13. Cannabinoid-Nano-Mizellen-Zubereitung nach Anspruch 1, **dadurch gekennzeichnet, dass** die Zubereitung ein Trockenpulver-Inhalationsmittel ist.

14. Verfahren für die Zubereitung der Cannabinoid-Nano-Mizellen-Zubereitung nach einem der Ansprüche 1 bis 13, wobei das Verfahren die folgenden Schritte umfasst:
(1) Zubereiten einer Cannabinoid-Nano-Mizellen-Lösung aus Cannabinoid und einem amphiphilen Polymer;
(2) Verdünnen der in Schritt (1) erhaltenen Mizellenlösung und Trocknen, um Cannabinoid-Nano-Mizellen-Pulver zu erhalten; und
(3) Zubereiten des in Schritt (2) erhaltenen Cannabinoid-Nano-Mizellen-Pulvers als die Cannabinoid-Nano-Mizellen-Zubereitung;
wobei Schritt (1) vorzugsweise die folgenden Schritte umfasst:
(1-1) Hinzufügen von Rohstoffkomponenten der Cannabinoid-Nano-Mizellen-Zubereitung nach einem der Ansprüche 1 bis 9 in ein Lösungsmittel zum Quellen;
(1-2) Scheren des in dem Schritt (1-1) erhaltenen Gemischs unter Verwendung eines Hochscher-Dispersionsemulgators, um eine Emulsion zu erhalten; und
(1-3) Rühren der in dem Schritt (1-2) erhaltenen Emulsion, um eine klare, transparente Nano-Mizellen-Lösung zu erhalten.

## Revendications

1. Préparation de nanomicelles de cannabinoïde, comprenant un cannabinoïde et un polymère amphiphile ; la teneur en cannabinoïde étant comprise entre 1 et 40 % en poids, et la teneur en polymère amphiphile étant comprise entre 1 et 99 %, et un procédé de préparation de la préparation comprenant les étapes consistant à :
(1) préparer une solution de nanomicelles de cannabinoïdes à partir de cannabinoïde et d'un polymère amphiphile ;
(2) diluer la solution micellaire obtenue à l'étape (1) et sécher pour obtenir une poudre de nanomicelles de cannabinoïde ; et
(3) préparer la poudre de nanomicelles de cannabinoïde obtenue à l'étape (2) sous forme d'une préparation de nanomicelles de cannabinoïde ;
le polymère amphiphile étant choisi parmi l'un ou une combinaison de deux ou plus de succinate de vitamine E polyéthylèneglycol, polyuréthane amphiphile, poloxamère, un copolymère tribloc acide polylactique-polyéthylèneglycol-acide polylactique, copolymère à blocs polyéthylèneglycol-acide polyacrylique, copolymère à blocs polyéthylèneglycol-acide polyaspartique, copolymère à blocs polyéthylèneglycol-poly(acide lactique-co-glycolique), copolymère à blocs polyéthylèneglycol-polycaprolactone, copolymère à blocs polyéthylèneglycol-acide polylactique et copolymère à blocs polyéthylèneglycol-polystyrène ;
le séchage à l'étape (2) étant une lyophilisation par atomisation, le mode d'atomisation étant choisi parmi l'une ou une combinaison de deux ou plus d'une atomisation pneumatique, atomisation par pression, atomisation centrifuge et atomisation ultrasonique ; et la préparation étant une préparation pour inhalation.

2. Préparation de nanomicelles de cannabinoïde selon la revendication 1, **caractérisée en ce que** le cannabinoïde est choisi parmi l'un ou une combinaison de deux ou plus de produits purs de cannabidiol (CBD), cannabidivarine (CBDV), cannabigerol (CBG), cannabichromène (CBC), cannabinol (CBN), cannabidibutol (CBDB), cannabielsoin (CBE), cannabicyclol (CBL) et cannabinodiol (CBND) ; ou,
le cannabinoïde étant un extrait de cannabis et comprenant un ou une combinaison de deux ou plus de CBD, CBDV, CBG, CBC, CBN, CBDB, CBE, CBL et CBND.

3. Préparation de nanomicelles de cannabinoïde selon la revendication 1, **caractérisée en ce que** le succinate de vitamine E polyéthylèneglycol est choisi parmi TPGS 200, TPGS 238, TPGS 400, TPGS 600, TPGS 1000, TPGS 2000, TPGS 3400, TPGS 3500, TPGS 4000 et/ou TPGS 6000.

4. Préparation de nanomicelles de cannabinoïde selon la revendication 1, **caractérisée en ce que** le polyuréthane amphiphile est obtenu en copolymérisant alternativement un segment de chaîne de polyéthylèneglycol et une matière première comprenant du diisocyanate ; le polyuréthane amphiphile ayant une masse moléculaire moyenne en nombre comprise entre 1 000 et 50 000, et le segment de chaîne de polyéthylèneglycol ayant une masse moléculaire moyenne en nombre comprise entre 200 et 12 000 ; et le diisocyanate étant choisi dans le groupe constitué par le diisocyanate d'hexaméthylène, le diisocyanate de triméthyl-1,6-hexaméthylène, le diisocyanate d'isophorone, le diisocyanate de cyclohexanediméthylène, le diisocyanate de 4,4-dicyclohexylméthane et le diisocyanate de 1,4-cyclohexane.

5. Préparation de nanomicelles de cannabinoïde selon la revendication 1, **caractérisée en ce que** le poloxamère est choisi parmi un ou une combinaison de deux ou plus de poloxamère 188, poloxamère 338, poloxamère 407, poloxamère 124, poloxamère 215 et poloxamère 237 ;
dans le copolymère tribloc acide polylactique-polyéthylèneglycol-acide polylactique, une partie de polyéthylèneglycol ayant une masse moléculaire moyenne en nombre comprise entre 200 et 12 000, un rapport de bloc PEG à LA étant de 1:(5-50) ; et
copolymère à blocs polyéthylèneglycol-acide polyacrylique, le copolymère à blocs polyéthylèneglycol-acide polyaspartique, le copolymère à blocs polyéthylèneglycol-poly(acide lactique-co-glycolique), le copolymère à blocs polyéthylèneglycol-polycaprolactone, le copolymère à blocs polyéthylèneglycol-acide polylactique et le copolymère à blocs polyéthylèneglycol-polystyrène ayant une masse moléculaire moyenne en nombre comprise entre 500 et 50 000, le polyéthylèneglycol étant l'éther monométhylique du polyéthylèneglycol, et la partie polyéthylèneglycol ayant une masse moléculaire moyenne en nombre comprise entre 200 et 12 000.

6. Préparation de nanomicelles de cannabinoïde selon la revendication 1, **caractérisée en ce que** la préparation de nanomicelles de cannabinoïde comprend en outre un agent protecteur de lyophilisation pharmaceutiquement acceptable, la teneur de l'agent protecteur de lyophilisation étant comprise entre 1 et 10 %, et l'agent protecteur de lyophilisation étant choisi parmi lactose, mannitol, sorbitol, cyclodextrine, hydroxypropyl-P-cyclodextrine, EDTA-2Na, tréhalose, glucose, xylitol et/ou maltose.

7. Préparation de nanomicelles de cannabinoïde selon la revendication 6, **caractérisée en ce que** la préparation de nanomicelles de cannabinoïde comprend en outre un régulateur de pH, la teneur en régulateur de pH étant comprise entre 0,01 et 10 %, et le régulateur de pH étant choisi parmi acide citrique, citrate de sodium, acide tartrique, tartrate de sodium, acide acétique, acétate de sodium, dihydrogénophosphate de sodium, hydrogénophosphate de disodium, acide chlorhydrique, acide lactique et/ou hydroxyde de sodium.

8. Préparation de nanomicelles de cannabinoïde selon l'une quelconque des revendications 1 à 7, **caractérisée en ce que** l'étape (1) comprend les étapes consistant à :
(1-1) ajouter des composants de matière première de la préparation de nanomicelles de cannabinoïde dans un solvant pour les faire gonfler ;
(1-2) cisailler le mélange obtenu à l'étape (1-1) à l'aide d'un émulsifiant dispersant à cisaillement élevé pour obtenir une émulsion ; et
(1-3) agiter l'émulsion obtenue à l'étape (1-2) pour obtenir une solution de nanomicelles transparente et claire.

9. Préparation de nanomicelles de cannabinoïde selon la revendication 8, **caractérisée en ce que** le rapport de masse entre le solvant et les composants de matière première à l'étape (1-1) est (2-10):1 ;
le gonflement à l'étape (1-1) est effectué pendant 0,1 à 2 h ;
le cisaillement à l'étape (1-2) est effectué pendant 1 à 30 min ; et
l'agitation à l'étape (1-3) étant effectuée de 15 à 35 °C.

10. Préparation de nanomicelles de cannabinoïde selon la revendication 8, **caractérisée en ce que** la solution de nanomicelles de cannabinoïdes a une taille de particules comprise entre 1 et 500 nm.

11. Préparation de nanomicelles de cannabinoïde selon l'une quelconque des revendications 1 à 7, **caractérisée en ce qu'**un taux de dilution à l'étape (2) est compris entre 2 et 100.

12. Préparation de nanomicelles de cannabinoïde selon la revendication 1, **caractérisée en ce que** la préparation est une préparation solide ou une préparation semi-solide.

13. Préparation de nanomicelles de cannabinoïde selon la revendication 1, **caractérisée en ce que** la préparation est une poudre sèche pour inhalation.

14. Procédé de préparation de la préparation de nanomicelles de cannabinoïde selon l'une quelconque des revendications 1 à 13, comprenant les étapes consistant à :
(1) préparer une solution de nanomicelles de cannabinoïdes à partir de cannabinoïde et d'un polymère amphiphile ;
(2) diluer la solution micellaire obtenue à l'étape (1) et sécher pour obtenir une poudre de nanomicelles de cannabinoïde ; et
(3) préparer la poudre de nanomicelles de cannabinoïde obtenue à l'étape (2) sous forme d'une préparation de nanomicelles de cannabinoïde ;
de préférence, l'étape (1) comprenant les étapes consistant à :
(1-1) ajouter des composants de matière première de la préparation de nanomicelles de cannabinoïde selon l'une quelconque des revendications 1 à 9 dans un solvant pour les faire gonfler ;
(1-2) cisailler le mélange obtenu à l'étape (1-1) à l'aide d'un émulsifiant dispersant à cisaillement élevé pour obtenir une émulsion ; et
(1-3) agiter l'émulsion obtenue à l'étape (1-2) pour obtenir une solution de nanomicelles transparente et claire.
